# EUROPEAN PATENT SPECIFICATION

(11) **EP 2 243 435 B1**
(45) Date of publication and mention of the grant of the patent: **01.04.2015**
(21) Application number: 10250806.6
(22) Date of filing: 21.04.2010
(51) Int. Cl.: A61B 17/34, A61B 17/00, A61B 19/00

(54) **Visual veress needle assembly**
Visuelle Veress-Nadel-Anordnung
Ensemble d'aiguille de Veress visuelle

(30) Priority: 22.04.2009 US 171610 P; 22.03.2010 US 728338
(43) Date of publication of application: 27.10.2010
(73) Proprietor: Covidien LP, Mansfield, MA 02048 (US)
(72) Inventor: Okoniewski, Gregory G., North Haven, CT 06473 (US)
(74) Representative: Soames, Candida Jane

(56) References cited:
- WO-A1-2008/079373
- WO-A2-2008/027448
- DE-A1- 19 957 785
- US-A1- 2005 288 622
- US-A1- 2008 262 302

## Description

### BACKGROUND

### 1. Technical field

The present disclosure relates to veress needles. More particularly, the present disclosure relates to control of veress needles used for surgical incision.

### 2. Background Of Related Art

During various surgical procedures it is often desirable to access areas within the body in a relatively non-invasive manner. In laparoscopic or endoscopic surgeries, a small incision is formed in the body to allow passage of various surgical instrumentation. Often these incisions are formed using a hollow pointed needle also referred to as a veress needle.

In some specific surgeries, such as, for example, hernia repair surgery, a small incision is made through the abdominal wall to access tissue within the abdominal cavity. Some such procedures require insufflation of the abdominal cavity to provide an operative space. This is typically accomplished with the insertion of a cannula through the incision. Additional incisions may be made through the abdominal wall to accommodate additional cannulas and surgical instrumentation.

Occasionally, the surgical instruments used to form the incision result in tears or nonuniform areas around the incision making it difficult to seal about the incision for proper insufflation. Additionally, there is a risk of over insertion resulting in penetration and damage to underlying anatomical structure.

WO2008/027448 discloses the features of the preamble of claim 1 and describes a surgical assembly in which a cannula is placed into an incision and then an endoscope is passed into the cannula to view the patient cavity.

DE19957785 describes a laparoscope comprising a hollow needle provided with optical fibres. A camera is provided to provide a view downwards from the distal end of the device of the patient's body cavity.

### SUMMARY

To advance the state of the art of surgical incision, the present disclosure relates to an optical system for use with a veress needle to view the incision and underlying anatomical structures. The present disclosure relates also to a veress needle and optical system capable of viewing in multiple directions within a body cavity.

According to the invention there is provided a visual veress needle assembly as set forth in the independent claim with preferred features recited in the dependent claims.

There is disclosed a visual veress needle assembly for use in viewing penetration of tissue and underlying anatomical structures. The visual veress needle assembly generally includes an optically conducting veress needle having a body portion with a hollow interior and a tissue penetrating distal tip and a stylet, the stylet being positionable through the hollow interior of the body portion of the needle. The stylet has a body portion that includes image transmitting structure incorporated therein, and a distal tip portion having a lens.

In one embodiment, the image transmitting structure of the body portion of the stylet is formed of optical fibers. In alternative contemplated embodiments, the body portion of the stylet incorporates optical chip technology.

In a specific embodiment, the lens is a wide angle lens. In an alternative embodiment, the wide angle lens is a fish eye lens. In yet a further embodiment, the lens is a directional lens.

In a particular embodiment, a distal portion of the stylet is flexible to view surrounding areas. The distal portion of the stylet is capable of articulating substantially 180° relative to a centerline axis of the hollow interior of the veress needle.

The distal portion of the stylet incorporates a shape memory material to assist in orienting the lens relative to the surrounding tissue. The shape memory material has a generally J-shape in an unstressed condition allowing the lens to be oriented up to 180° relative to the veress needle, in a particular embodiment.

In one embodiment, the tissue penetrating tip of the veress needle is formed at a predetermined angle relative to the hollow body portion to facilitate slicing through tissue.

In a further embodiment, a connector is disposed at a proximal end of the stylet such that the connector conveys optical data passing through the stylet to external imaging devices.

In one embodiment, the optically conductive veress needle includes a proximal end and a distal end. The proximal end and the distal end each have a translucent surface enabling transmission of light through the body portion of the veress needle from the proximal end to the distal end. The visual veress needle assembly may further include a light source disposed in the vicinity of the proximal end of the veress needle. The light source may be configured and disposed to emit light onto the translucent surface at the proximal end such that the light passes through the body portion of the veress needle and is emitted at the translucent surface at the distal end.

The optically conductive veress needle may be made from a translucent material. The translucent material may be selected from a group consisting of a resin, a plastic and an oxide. The plastic may be a polycarbonate and the oxide is one of a group consisting of glass and ceramic.

### DESCRIPTION OF THE DRAWINGS

Various embodiments of the presently disclosed visual veress needle assembly are disclosed herein with reference to the drawings, wherein:
FIG. 1 is a side view, partially shown in section, of one embodiment of a visual veress needle assembly, including a veress needle and stylet, inserted through tissue;
FIG. 1A is a cross-section of the veress needle and stylet of FIG. 1;
FIG. 2 is a top view of the visual veress needle assembly of FIG. 1;
FIG. 3 is a side view of the visual veress needle assembly of FIG. 1;
FIG. 4 is a side view of an alternate tip configuration of a stylet for use with a veress needle;
FIG. 5 is a side view, shown in section, an articulating mechanism of the stylet incorporating an articulation mechanism;
FIG. 6 is a side view, shown in section, of the stylet of FIG. 5 in the articulated position;
   and
FIG. 7 is a side view, partially shown in section, of the veress needle assembly of FIG. 1 with a valve assembly and inserted through tissue.

### DETAILED DESCRIPTION OF EMBODIMENTS

Embodiments of the presently disclosed visual veress needle assembly will now be described in detail with reference to the drawings wherein like numerals designate identical or corresponding elements in each of the several views. As is common in the art, the term 'proximal" refers to that part or component closer to the user or operator, i.e. surgeon or physician, while the term "distal" refers to that part or component further away from the user.

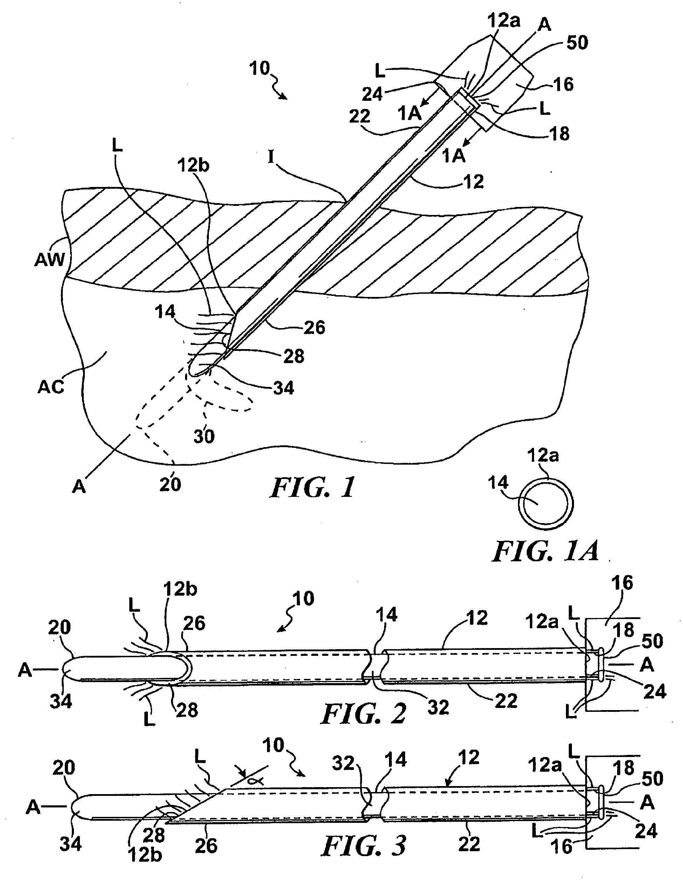

Referring to FIG. 1, there is disclosed a visual veress needle assembly 10 for use in creating an incision **I** through a tissue, such as, for example abdominal wall **AW.** Visual veress needle assembly 10 allows a surgeon to view visual veress needle 12 as it passes through abdominal wall **AW** as well as viewing an operative site, for example, abdominal cavity **AC** within the body of a patient. Visual veress needle assembly 10 generally includes an optically conducting veress needle 12 and an optical insert or stylet 14 insertable through needle 12. A connector 16 is provided at a proximal end 18 of stylet 14 to connect stylet 14 to various viewing, optical or electrical conversion or recording equipment, such as cameras, viewing screens, computerized data analysis devices, recording equipment, etc. The optically conducting veress needle 12 is made from glass, ceramic, polycarbonate or other suitable substantially clear or translucent resin/plastic, oxide or similar suitable material. As illustrated in FIG. 1, a light source 50 is provided internally within the connector 16 as at least part of the viewing, optical or electrical conversion or recording equipment included within the connector 16. A distal end or tip 20 of stylet 14 is formed of a viewing device described in more detail hereinbelow.

Referring now to FIGS. 1-3, veress needle 12 generally includes a hollow body portion 22 having an open proximal end 24 for receipt of stylet 14. As illustrated in FIG. 1A, at the proximal end 24, a concentrically circular translucent surface 12a is formed in the veress needle 12. An open distal end 26 of body portion 22 is formed with a sharp tissue penetrating tip 28. Tip 28 is formed at an angle α relative to body portion 22 to facilitate slicing through abdominal wall **AW** (FIG. 3). In the exemplary embodiment of the visual veress needle assembly 10 illustrated in FIGS. 1, 1A and 2-7, since the tip 28 is formed at angle α relative to the body portion 22, an oval translucent surface 12b is formed in the veress needle 12 at the distal end 26. To enhance the transmission of light **L** emitted from the light source 50, the light source 50 may be configured in a circular or tubular configuration similar to a circular fluorescent bulb and disposed so as to maximize the intensity of light **L** emitted from the light source 50 onto the surface 12a of the veress needle 12. Light **L** emitted from the light source 50 passes through the surface 12a and travels through the body portion 22 of the veress needle 12 and is then emitted from the oval surface 12b at the distal end 26 to illuminate the incision **I** and underlying anatomical structures within the abdominal cavity **AC.**

Thus, the optically conductive veress needle 12 includes proximal end 24 and distal end 26. The proximal end 24 and the distal end 26 each have a translucent surface 12a and 12b, respectively, enabling transmission of light **L** through the body portion 22 of the veress needle 12 from the proximal end 24 to the distal end 26. The light source 50 may be disposed in the vicinity of the proximal end 24 of the veress needle 12. The light source 50 may be configured and disposed to emit light **L** onto the translucent surface 12a at the proximal end 24 such that the light **L** pa sses through the body portion 22 of the veress needle 12 and is emitted at the translucent surface 12b at the distal end 26.

As noted above, stylet 14 is provided to allow a surgeon to view veress needle 12 as it forms incision I through abdominal wall **AW,** as well as viewing abdominal cavity **AC** including underlying anatomical structures (not shown) to prevent damaging those structures during insertion of visual veress needle 10. A distal portion 30 of stylet 14 is flexible or articulating to view incision **I,** surrounding tissues or the insertion of additional devices through abdominal wall **AW** as described in more detail hereinbelow (FIG. 1).

In one embodiment, stylet 14 includes a body portion 32 containing or substantially constructed from image and light carrying fiber optical materials. Proximal end 18 of stylet 14 is connected to connector 16 so as to pass the data transmitted through body portion 32 to devices enabling the surgeon to view the operative site. It should be noted that, while in the present embodiment, body portion 32 is formed from optical fibers, other means of obtaining and transmitting optical data are also contemplated. For example, while not specifically shown, an optical system including optical chip technology may be provided.

Tip 20 includes a lens 34 for obtaining an optical image. In this embodiment, lens 34 is a wide angle or fish eye type lens for maximizing the area viewed. The use of wide angle lens 34 also assists in being able to view proximally back toward incision **I.**

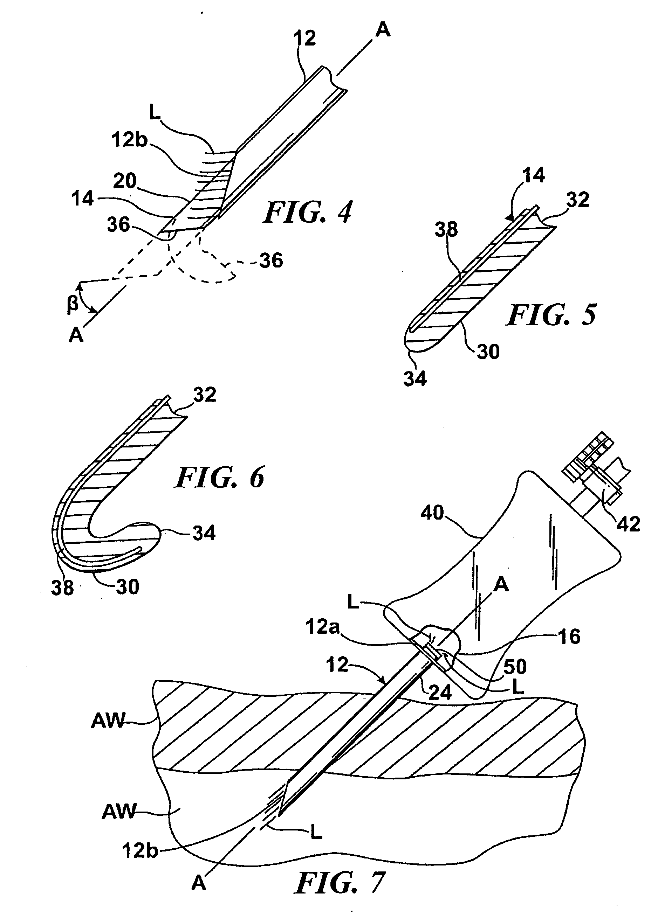

Referring for the moment to FIG. 4, there is disclosed an alternative lens configuration provided on distal tip 20 of stylet 14. Tip 20 is formed with a generally flat lens 36. Lens 36 is provided to view in a relatively narrow, generally singular direction to isolate, and maximize the image of, particular areas of the operative site. Lens 36 is formed at an angle P relative to body portion 32 of stylet 14 and relative to longitudinal centerline A-A of the veress needle 12. By articulating and/or rotating distal portion 30 of stylet 14, lens 36 may be directed at the specific area to be viewed.

Referring now to FIGS. 5 and 6, as noted hereinabove, distal portion 30 of stylet is flexible so as to orient lens 34, or lens 36, in a particular desired direction. Various mechanisms are contemplated to accomplish the articulation, such as for, example, cables, springs, linkages, pneumatics, hydraulics, etc. In this embodiment, motion is provided by the incorporation of a length of shape memory material 38 extending at least partially through body portion 32 and distal portion 30 of stylet 14. Shape memory material 38 is formed into a hook or J-shape in the unstressed condition.

With reference to FIGS. 1 and 5, when distal portion 30 of stylet 14 is retracted within hollow body portion 22 of veress needle 12, shape memory material 38, and thus distal portion 30 are constrained to a relatively straight configuration. As shown in FIGS. 1 and 6, as distal portion 30 of stylet is advanced beyond tissue penetrating tip 28 of veress needle 12, shape memory material 38 is no longer constrained and returns to the unstressed, generally J-shape configuration. Since shape memory material 38 is embedded within distal portion 30, return of shape memory material 38 to the unstressed configuration moves flexible distal portion with it. Thus, depending on the degree of advancement of distal portion 30 beyond tip 28 of veress needle 14, the angle or orientation of lens 34 on distal portion 30 relative to veress needle 14 is controlled. While not specifically shown, rotation of stylet 14 within veress needle 12 also serves to direct lens 34. Therefore, the combination of articulation and rotational capabilities allows a surgeon to view the entire area within abdominal cavity **AC.**

In a specific embodiment, advancement of distal portion 30 a predetermined amount relative to veress needle 12 results in lens 34 being oriented from approximately or substantially zero degrees (0°) to approximately or substantially one hundred eighty degrees (180°) relative to centerline axis A-A of the veress needle 12 (see FIGS. 1-4 and 7) to view incision **I** or other areas of abdominal wall **AW** to view the insertion of additional instrumentation through abdominal wall **AW**.

Finally, with reference to FIG. 7, once stylet 14 has been used to observe the passage of visual veress needle 10 through abdominal wall **AW,** it may, as noted above, be oriented and utilized to view areas within abdominal cavity **AC.** Once visualization is complete, stylet 14 may be removed from within hollow body portion 22 of veress needle 12. Veress needle 12 may be left in place through incision **I** and used as a conduit for passage of insufflation gasses or other operative surgical instrumentation. For example, a cannula body 40 having a valve 42 may be attached to proximal end 24 of body portion 22 to provide insufflation gasses into abdominal cavity **AC.** Alternatively, veress needle 12 may act as a guide for a cannula system having an inflation tube where the inflation tube is incapable of penetrating tissue. In this instance, veress needle 12 may be left in place or subsequently removed.

Thus, visual veress needle assembly 10 provides a means of penetrating tissue and visualizing the penetration as visual veress needle 12 passes through tissue. Additionally, underlying anatomical structures may be identified and observed during penetration to avoid or reduce the risk of injury to such structures. Further, the penetrations of additional tissue penetrating trocars may be observed, as well as locating additional penetration sites.

It will be understood that various modifications may be made to the embodiments disclosed herein. For example, as noted above, other optical system are contemplated for incorporation into the stylet, for example, optical chip technology, fluid visualization systems, etc. Further, the disclosed visual veress needle may incorporate other detection system including infrared or thermal or radiation detection capabilities. Additionally, other lens shapes are also contemplated, such as, for example telephoto or zoom lens, macro lenses, etc. Therefore, the above description should not be construed as limiting, but merely as exemplifications of particular embodiments. Those skilled in the art will envision other modifications within the scope of the claims appended hereto.

## Claims

1. A visual veress needle assembly (10) for viewing penetration of tissue and underlying anatomical structures comprising:
an optically conducting veress needle (12) having a body portion with a hollow interior and a tissue penetrating distal tip (28); and
a stylet (14), the stylet being positionable through the hollow interior of the body portion of the needle, the stylet having a body portion including an image transmitting structure incorporated therein, to allow, in use, a surgeon to view the veress needle as it forms an incision,
**characterised in that**
the distal portion of the stylet incorporates a shape memory material such that a distal portion of the stylet is flexible or articulating to view the incision or surrounding tissues when extended out of the tissue penetrating distal tip.

2. The visual veress needle assembly as recited in claim 1, wherein the image transmitting structure of the body portion (32) of the stylet is formed of optical fibers.

3. The visual veress needle assembly as recited in claim 1 or claim 2, further comprising a lens (34) at a distal tip portion of the stylet.

4. The visual veress needle assembly as recited in claim 3, wherein the lens is a wide angle lens.

5. The visual veress needle assembly as recited in claim 3, wherein the lens is a directional lens.

6. The visual veress needle assembly as recited in any of the preceding claims, wherein the distal portion of the stylet (20) is capable of articulating substantially 180° relative to a centerline axis of the hollow interior of the veress needle.

7. The visual veress needle assembly as recited in any preceding claim, wherein the shape memory material has a generally J-shape in an unstressed condition.

8. The visual veress needle assembly as recited in any preceding claim, wherein the tissue penetrating tip of the veress needle is formed at a predetermined angle relative to the hollow body portion.

9. The visual veress needle assembly as recited in any preceding claim, further comprising a connector (16) disposed at a proximal end of the stylet, wherein the connector conveys optical data passing through the stylet to external imaging devices.

10. The visual veress needle assembly as recited in any preceding claim, wherein the optically conductive veress needle includes a proximal end (24) and a distal end (26), the proximal end and the distal end each having a translucent surface (12b) enabling transmission of light through the body portion of the veress needle from the proximal end to the distal end.

11. The visual veress needle assembly as recited in claim 10, further comprising a light source (50) disposed in the vicinity of the proximal end of the veress needle, the light source configured and disposed to emit light onto the translucent surface at the proximal end such that the light passes through the body portion of the veress needle and is emitted at the translucent surface at the distal end.

12. The visual veress needle assembly as recited in any preceding claim, wherein the veress needle is made from a translucent material.

13. The visual veress needle assembly as recited in claim 12, wherein the translucent material is one of the group consisting of a resin, a plastic and an oxide, preferably wherein the plastic is polycarbonate and the oxide is one of the group consisting of glass and ceramic.

## Patentansprüche

1. Veres-Sichtkanülen-Baugruppe (10) zum Einsehen der Gewebepenetration und darunter liegender anatomischer Strukturen, wobei die Baugruppe Folgendes aufweist:
eine optisch leitende Veres-Kanüle (12) mit einem Körperabschnitt mit Innenhohlraum und einer distalen, Gewebe penetrierenden Spitze (28); und
einen Mandrin (14), wobei der Mandrin durch den Innenhohlraum des Körperabschnitts der Kanüle positionierbar ist und wobei der Mandrin einen Körperabschnitt mit darin integrierter bildübertragender Struktur aufweist, um es einem Chirurgen bei der Verwendung zu ermöglichen, die Veres-Kanüle bei der Ausführung eines Einschnitts einzusehen,
**dadurch gekennzeichnet, dass** im distalen Abschnitt des Mandrins ein Formspeicher-Material integriert ist, sodass ein distaler Abschnitt des Mandrins flexibel bzw. gelenkbewehrt ist, um den Einschnitt bzw. die umgebenden Gewebe beim Austritt an der das Gewebe penetrierenden distalen Spitze einzusehen.

2. Veres-Sichtkanülen-Baugruppe nach Anspruch 1, wobei die bildübertragende Struktur des Körperabschnitts (32) des Mandrins aus optischen Fasern gebildet ist.

3. Veres-Sichtkanülen-Baugruppe nach Anspruch 1 oder 2, die weiter ein Objektiv (34) an einem distalen Spitzenabschnitt des Mandrins aufweist.

4. Veres-Sichtkanülen-Baugruppe nach Anspruch 3, wobei das Objektiv ein Weitwinkelobjektiv ist.

5. Veres-Sichtkanülen-Baugruppe nach Anspruch 3, wobei das Objektiv ein Richtobjektiv ist.

6. Veres-Sichtkanülen-Baugruppe nach einem der vorstehenden Ansprüche, wobei sich der distale Abschnitt des Mandrins (20) relativ zu einer Mittelachse des Innenhohlraums der Veres-Kanüle in im Wesentlichen 180° positionieren kann.

7. Veres-Sichtkanülen-Baugruppe nach einem der vorstehenden Ansprüche, wobei das Formspeicher-Material eine im unbelasteten Zustand im Allgemeinen J-förmige Form aufweist.

8. Veres-Sichtkanülen-Baugruppe nach einem der vorstehenden Ansprüche, wobei die Gewebe penetrierende Spitze der Veres-Kanüle in einem vorbestimmten Winkel relativ zum Innenhohlraum-Abschnitt ausgebildet ist.

9. Veres-Sichtkanülen-Baugruppe nach einem der vorstehenden Ansprüche, die weiter einen an einem proximalen Ende des Mandrins angeordneten Anschluss (16) aufweist, wobei der Anschluss optische Daten, die den Mandrin passieren, zu externen bildgebenden Einrichtungen übermittelt.

10. Veres-Sichtkanülen-Baugruppe nach einem der vorstehenden Ansprüche, wobei die optisch leitende Veres-Kanüle ein proximales Ende (24) und ein distales Ende (26) aufweist, wobei das proximale Ende und das distale Ende jeweils eine transluzente Oberfläche (12b) aufweisen, die die Lichtübertragung durch den Körperabschnitt der Veres-Kanüle vom proximalen Ende zum distalen Ende ermöglicht.

11. Veres-Sichtkanülen-Baugruppe nach Anspruch 10, die weiter eine Lichtquelle (50) aufweist, die in der Nähe des proximalen Endes der Veres-Kanüle angeordnet ist, wobei die Lichtquelle konfiguriert und eingerichtet ist, um Licht am proximalen Ende auf die transluzente Oberfläche zu emittieren, sodass das Licht den Körperabschnitt der Veres-Kanüle passiert und am distalen Ende an der transluzenten Oberfläche emittiert wird.

12. Veres-Sichtkanülen-Baugruppe nach einem der vorstehenden Ansprüche, wobei die Veres-Kanüle aus einem transluzenten Material hergestellt ist.

13. Veres-Sichtkanülen-Baugruppe nach Anspruch 12, wobei das transluzente Material ein Material der Gruppe ist, die ein Harz, einen Kunststoff und ein Oxid aufweist, und wobei der Kunststoff vorzugsweise Polycarbonat ist und das Oxid vorzugsweise ein Oxid der Gruppe bestehend aus Glas und Keramik ist.

## Revendications

1. Ensemble d'aiguille de Veress visuelle (10) pour observer la pénétration d'un tissu et de structures anatomiques sous-jacentes comprenant :
une aiguille de Veress optiquement conductrice (12) ayant une partie de corps avec un intérieur creux et une pointe distale (28) pénétrant dans le tissu ; et
un stylet (14), le stylet pouvant être positionné à travers l'intérieur creux de la partie de corps de l'aiguille, le stylet ayant une partie de corps comprenant une structure transmettrice d'image qui y est incorporée afin de permettre, à l'usage, à un chirurgien d'observer l'aiguille de Veress lorsqu'elle forme une incision,
**caractérisé en ce que** :
la partie distale du stylet incorpore un matériau à mémoire de forme tel qu'une partie distale du stylet soit souple ou s'articule pour observer l'incision ou les tissus environnants lorsqu'il se déploie de la pointe distale pénétrant dans le tissu.

2. Ensemble d'aiguille de Veress visuelle selon la revendication 1, dans lequel la structure transmettrice d'image de la partie de corps (32) du stylet est formée de fibres optiques.

3. Ensemble d'aiguille de Veress visuelle selon la revendication 1 ou la revendication 2, comprenant en outre une lentille (34) à une partie de pointe distale du stylet.

4. Ensemble d'aiguille de Veress visuelle selon la revendication 3, dans lequel la lentille est une lentille à angle large.

5. Ensemble d'aiguille de Veress visuelle selon la revendication 3, dans lequel la lentille est une lentille directionnelle.

6. Ensemble d'aiguille de Veress visuelle selon l'une quelconque des revendications précédentes, dans lequel la partie distale du stylet (20) est à même de s'articuler sensiblement sur 180° par rapport à un axe central de l'intérieur creux de l'aiguille de Veress.

7. Ensemble d'aiguille de Veress visuelle selon l'une quelconque des revendications précédentes, dans lequel le matériau à mémoire de forme a une forme générale en J à l'état non contraint.

8. Ensemble d'aiguille de Veress visuelle selon l'une quelconque des revendications précédentes, dans lequel la pointe de l'aiguille de Veress pénétrant dans le tissu est formée sous un angle prédéterminé par rapport à la partie de corps creuse.

9. Ensemble d'aiguille de Veress visuelle selon l'une quelconque des revendications précédentes, comprenant en outre un connecteur (16) disposé à une extrémité proximale du stylet, dans lequel le connecteur achemine des données optiques passant à travers le stylet à des dispositifs d'imagerie externes.

10. Ensemble d'aiguille de Veress visuelle selon l'une quelconque des revendications précédentes, dans lequel l'aiguille de Veress optiquement conductrice comprend une extrémité proximale (24) et une extrémité distale (26), l'extrémité proximale et l'extrémité distale ayant chacune une surface translucide (12b) permettant la transmission de lumière à travers la partie de corps de l'aiguille de Veress de l'extrémité proximale à l'extrémité distale.

11. Ensemble d'aiguille de Veress visuelle selon la revendication 10, comprenant en outre une source de lumière (50) disposée au voisinage de l'extrémité proximale de l'aiguille de Veress, la source de lumière étant configurée et disposée pour émettre de la lumière sur la surface translucide à l'extrémité proximale de sorte que la lumière passe à travers la partie de corps de l'aiguille de Veress et soit émise sur la surface translucide à l'extrémité distale.

12. Ensemble d'aiguille de Veress visuelle selon l'une quelconque des revendications précédentes, dans lequel l'aiguille de Veress est constituée d'un matériau translucide.

13. Ensemble d'aiguille de Veress visuelle selon la revendication 12, dans lequel le matériau translucide est l'un des matériaux du groupe constitué des suivants : une résine, une matière plastique et un oxyde, de préférence dans lequel la matière plastique est un polycarbonate et l'oxyde est l'un des matériaux du groupe constitué du verre et de la céramique.
